(19) Europäisches Patentamt
European Patent Office
Office européen des brevets

(11) Veröffentlichungsnummer: **0 114 387 B1**

(12) **EUROPÄISCHE PATENTSCHRIFT**

(45) Veröffentlichungstag der Patentschrift: **05.02.86**

(51) Int. Cl.⁴: **C 07 C 129/12**, A 61 K 31/155, A 01 N 47/44

(21) Anmeldenummer: **83113016.6**

(22) Anmeldetag: **23.12.83**

# (54) Salze von Bis-Guanidin-Verbindungen, Verfahren zu ihrer Herstellung und ihre Verwendung als mikrobizide Mittel.

(30) Priorität: **28.12.82 DE 3248342**

(43) Veröffentlichungstag der Anmeldung: **01.08.84 Patentblatt 84/31**

(45) Bekanntmachung des Hinweises auf die Patenterteilung: **05.02.86 Patentblatt 86/6**

(84) Benannte Vertragsstaaten: **AT BE CH DE FR GB IT LI NL SE**

(56) Entgegenhaltungen: **CHEMICAL ABSTRACTS, Band 64, Nr. 4, 14. Februar 1966, Spalte 4954d, Columbus, Ohio, USA**

(73) Patentinhaber: **HOECHST AKTIENGESELLSCHAFT, Postfach 80 03 20, D-6230 Frankfurt am Main 80 (DE)**

(72) Erfinder: **Blaschke, Günter, Dr., Bajuwarenstrasse 36, D-8261 Winhöring (DE)**
Erfinder: **May, Adolf, Dr., Dahlienweg 5, D-6238 Hofheim am Taunus (DE)**
Erfinder: **Bücking, Hans-Walter, Dr., In den Padenwiesen 30, D-6233 Kelkheim (Taunus) (DE)**
Erfinder: **Wallhäusser, Karl-Heinz, Prof. Dr., Lessingstrasse 20, D-6238 Hofheim am Taunus (DE)**

**EP 0 114 387 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung bezieht sich auf neue Guanidiniumsalze, ein Verfahren zu deren Herstellung und die Verwendung dieser Stoffe in mikrobiziden Zusammensetzungen.

Es ist seit langem bekannt, daß Alkylguanidine, Alkyldiguanidine und deren Salze Verbindungen mit guter bakterizider und fungizider Wirkung darstellen (siehe DE-PS 679 787; DE-PS 1 249 457; K.H. Wallhäußer 'Sterilisation - Desinfektion - Konservierung', Georg Thieme Verlag Stuttgart, 1978, Seite 363 ff.)- Wegen der besehränkten Löslichkeit dieser Verbindungen in Wasser und ihrer teilweise schlechten Verträglichkeit mit hartem und kochsalzhaltigem Wasser ist deren Einsatzmöglichkeit jedoch bedrenzt. Ein Zusatz von löslichkeits- und stabilitätsverbessernden Mitteln, wie beispielsweise von guaternären Ammonium- oder Phosphoniumverbindungen oder von Fettalkylaminsalzen, beeinträchtigt wiederum die mikrobizide Wirkung.

Gemäß der DE-OS 28 08 865 sollen diese Nachteile dadurch vermieden werden, daß man Alkyldiguanidinium-Salze mit Polyoxethylen-Polyoxpropylen-Blockmischpolymerisaten abmischt. Wegen des Gehalts solcher Mittel an diesen Polyoxalkylaten wird jedoch der Wirkstoffanteilzwangsläufig vermindert, so daß keine hochkonzentrierten Einsteliungcn erhalten werden können.

Somit besteht die Aufgabe, nach Guanidin-Derivaten zu suchen, die in Wasser hinreichend löslich sind und solche Zusätze nicht erfordern. Gleichzeitig gibt es ein Bedürfnis nach mikrobiziden Präparaten dieser Art mit unterschiedlicher Konstitution und hoher Wirksamkeit, um Resistenzbildungen, wie sie bei längerem Einsatz des gleichen Mittels auftreten können, zu vermeiden.

Zur Lösung dieser Aufgabe stellt die Erfindung Salze von Bis-Guanidin-Derivaten der allgemeinen Formel

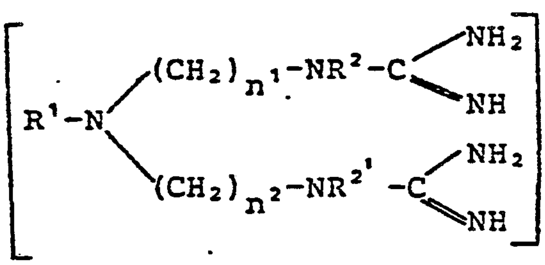

•a HX (I)

zur Verfügung, worin $R^1$ einen Alkylrest von 8 bis 18 C-Atomen, einen olefinisch ungesättigten Kohlenwasserstoff-Rest der gleichen Kettenlänge und mit 1 bis 3 Doppelbindungen, einen oxalkylierten Alkylrest der Formel $R^{11}(OCHR^1CH_2)m$, worin $R^{11}$die gleiche Bedeutung wie $R^1$ hat und in der m (als Mittelwert) eine Zahl von 1 bis 10 bedeutet, $R^1$ H oder $CH_3$ ist und bei Werten von m 1 auch beide Bedeutungen haben kann, worin ferner $R^2$ und $R^2$, gleich oder verschieden, Wasser-stoff, einen Alkylrest mit 1 bis 3 C-Atomen oder einen Hydroxyalkylrest mit 1 bis 3 C-Atomen bedeuten, $n^1$ und $n^2$, gleich oder verschieden, die Zahlen 2 oder 3 sind und HX eine zur Salzbildung mit dem Bis-Guanidin befähigte Säure sowie a eine Zahl von 1 bis 3 ist.

Bevorzugt ist in dieser Formel $R^1$ ein Alkylrest mit 8 bis . 18 C-Atomen, $R^{1'}$ Wasserstoff, $R^2$ und $R^{2'}$ Wasserstoff, $n^1 = n^2 = 3$ und $a = 2$. HX ist eine ein- oder mehrbasische anorganische oder organische Säure, die mit den genannten Bis-Guanidin-Derivaten Salze zu bilden vermag, vorzugsweise Schwefelsäure, Phosphorsäure, Salpetersäure, Chlorwasserstoff, Ameisensäure, Essigsäure, Milchsäure, Glykolsäure, Stearinsäure, Laurinsäure oder Ölsäure.

In diesen erfindungsgemäßen Salzen von Bis-GuanidinDerivaten der Formel I sind die Alkyl-oder Alkenylreste $R^1$, die dem primären Ausgangsamin entstammen, häufig Gemische oder Kettenschnitte bevorzugt mit der Kettenverteilung der Reste von natürlichen Fettsäuren, wie insbesondere der Kokos-, Talg- oder Palmkernfettsäure, aus denen diese Ausgangsamine, beispielsweise über die bekannten Wege der Nitrilhydrierung oder der Ammonolyse der entsprechenden Alkohole, gewonnen werden können. Die zur Herstellung der primären Amine mittels Ammonolyse verwendeten Alkohole können neben Fettalkoholen auch solche mit gerader oder verzweigter Kette aus dem Ziegler-Prozeß (Ethylen-Aufbaualkohole) oder aus der Oxosynthese sein. Desgleichen können mit Ethylenoxid oder Propylenoxid oder deren Gemischen kondensierte Fettalkohole oder entsprechende Ethernitrile bei der Ammonolyse oder Nitrilhydrierung als Ausgangsprodukte dienen.

Zur Herstellung der erfindungsgemäßen Verbindungen wird zunächst in bekannter Weise ein solches primäres Amin der Formel $R^1NH_2$, wobei $R^1$ die vor-genannte Bedeutung hat, mit 2 mol eines reaktionsfähigen Nitrils von 2 bis 3 C-Atomen (einschließlich der CN-Gruppe) oder Gemischen solcher Nitrile zu einer Verbindung der allgemeinen Formel

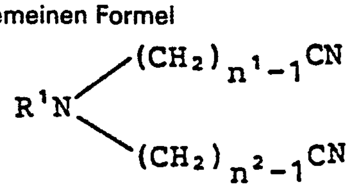

in einer Dicyanalkylierungsreaktion umgesetzt. Diese Reaktion ist bekannt, beispielsweise aus der US-PS 3 028 415. Sie kann sowohl mit saurer als auch mit basischer Katalyse, mit Hilfe von Lösungsmitteln, wie Wasser oder auch niederkettigen Alkoholen, drucklos oder unter erhöhtem Druck, kontinuierlich oder diskontinuierlich durchgeführt werden. Als saure Katalysatoren werden Essigsäure, Phosphorsäure, Salzsäure und andere Mineralsäuren genannt (US-PS 3 615 797, US-PS 3 028 415, DE-OS 19 41 913), als basische Katalysatoren sind empfohlen worden Natrium- oder Kaliumhydroxid,

Alkalialkoholate, Trimethylbenzylammoniumhydroxid und Morpholin (Kirk-Othmer, Encyclopedia of Chemical Technology, 1965,Band 6, Seite 634 ff.; H. A. Bruson 'Cyanoethylation', Organic Reactions, 5, 1949, Seite 79 ff., Verlag John Wiley and Sons, New York). Als Co-Katalysatoren oder auch als Lösungsvermittler werden Wasser oder niedere Alkohole, wie Methanol, Ethanol,Isopropanol oder Gemische derselben in Anteilen von 1 bis 20 Gew.-% zugegeben. Die Dicyanalkylierung wird unter Normaldruck oder leichtem bis mittlerem Überdruck von 1 bis 20 bar, gegebenenfalls in Gegenwart eines Inertgases, und bei Temperaturen von 60 bis 150 °C durchgeführt. Das Cyanalkylierungsmittel, vorzugsweise Acrylnitril oder Chloracetonitril, wird stöchiometrisch oder in einem bis zu vierfachem Überschuß angewandt.

Anschließend wird das so gewonnene Dicyanalkylierungsprodukt in Gegenwart von Wasserstoff zu einer Verbindung der Formel

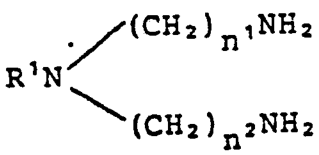 (II)

reduziert. Die Reduktion wird mit Raney-Nickel oder Raney-Kobalt oder aber mit Nickel- oder Kobalt-Trägerkatalysatoren durchgeführt, und zwar unter Einsatz von 1 bis 10 Gew.-% Katalysator, bevorzugt 1 bis 5 Gew.-% und bei Drücken von 50 bis 200 bar Wasserstoff und Temperaturen von 60 bis 150 °C; die Reaktionszeit beträgt dabei etwa 1 bis 5 Stunden. Gegebenenfalls wird das erhaltene Amin der Formel II mit höchstens 2 mol an Alkyloder Hydroxyalkylhalogenid mit einem Alkylrest von 1 bis 3 C-Atomen in an sich bekannter Weise umgesetzt. Entsprechend kann das Amin der Formel II mit Formaldehyd, Acetaldehyd oder Propionaldehyd kondensiert und anschließend zur Hydroxyalkylverbindung oder Alkylverbindung reduziert werden. Auf diese Weise erhält man Amine der Formel

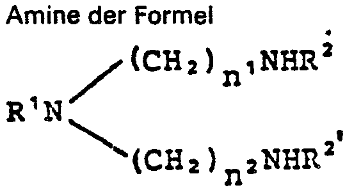 (II') ,

in der zumindest ainer der beiden Reste $R^2$ und $R^2$ oder beide Alkyl- oder Hydroxyalkylreste mit 1 bis 3 C-Atomen sind und im ersteren Fall der andere Wasserstoff ist.

Das so gewonnene Amin wird sodann mit Cyanamid $H_2N$-CN oder mit Dicyanamid $HN=C(NH_2)$-NHCN zusammen mit einer Säure HX in wäßriger Lösung, gegebenenfalls unter Zugabe von Alkanolen, zum entsprechenden Salz des jeweiligen Bis-Guanidin-Derivats der Formel I umgewandelt. Für die Umsetzung sind 2 mol Cyanamid oder 1 mol Dicyanamid erforderlich, vorzugsweise wird jedoch ein leichter Überschuß von bis zu 10 % angewendet. Desgleichen wird die die Protonierung des Bis-Guanidin-Derivats bewirkende Säure HX verzugsweise in leichtem Überschuß in bezug auf die äquavalente Menge eingesetzt, wobei die äquivalente Menge a dieser Säure so bemessen ist, daß - wie jeweils gewünscht - eine einfache, zweifache oder auch dreifache, vorzugsweise aber eine zweifache Protonierung des Bis-Guanidin-Derivats erfolgt, und zwar unter Berücksichtigung der ein-, zwei- oder dreibasischen Natur der jeweiligen Säure. Demgemäß ergibt sich bei mehrbasichen Säuren die einzusetzende Molmenge aus dem gewünschten Protonierungsgrad angeteilt durch die Basizität der jeweils angewandten Säure. Die erhaltenen Verbindungen können demzufolge auch Gemische solcher einfach, zweifach und dreifach protonierten Spezies darstellen, wobei a als Mittelwert dann auch gebrochene Werte annehmen kann. Die Säure und das Cyanamid oder Dicyanamid werden in beliebiger Reihenfolge nacheinander oder auch gleichzeitig zu dem Ausgangsamin hinzugetropft, wobei die Mischung etwa 3 bis 6 Stunden bei etwa 70 °C am Rückfluß erhitzt wird, und zwar solange, bis keine freien Cyanidionen mehr nachweisbar sind. Die erhaltenen wäßrigen oder wäßrig-alkoholischen Lösungen könncn gegebenenfalls direkt oder nach Aufkonzentrieiung Verwendung finden. Man kann die erfindungsgemäßen Verhindungen der Formel I aber auch als gut kristallisierte Substanzen gewinnen.

Die erfindungsgemäßen Verbindungen der Formel I sind hochwirksame Mikrobizide mit sehr guter bakterizider, fungizider und algizider Wirkung. Sie besitzen eine ausgezeichnete Wasserlöslichkeit und sind mit hartem Wasser und mit Salzwasser auch ohne Zusätze gut verträglich. Sie können in Form hochkonzentrierter Einstellungen in Wasser oder Alkoholen oder deren Gemischen mit bis zu 90 Gew.-% Wirkstoffgehalt gewichtssparcnd versandt und in den Handel gebracht werden, wobei solche Konzentrate dennoch bei Raumtemperatur flüssig sind. Der Mindestgehalt (vorzugsweise mindestens 10 Gew.-%) solcher Einstellungen ist nicht kritisch, da solche hochkonzentrierten Einstellungen vor der Anwendung problemlos mit Wasscr und/oder Alkohol auf den gewünschten Wirkstoffgehalt verdünnbar sind. Sie sind besonders geeignet zur Unterdrückung von Befall mit anaeroben sulfatreduzierenden Bakterien, insbesondere der Arten Desulfovibrio desulfuricans, die bei der Sekundärförderung des Erdöls auftreten und diese durch Korrosionsschäden an Leitungen und Behältern sowie durch Reduzierung der Permeabilität der Lagerstätten behindern. Da diese Bakterienarten in hohem Grade anpassungsfähig und resistenzbildend sind, ist es wichtig, das Bakterizid im Abstand von einigen Wochen zu wechseln. Hierzu stellt die Erfindung ein weiteres Präparat mit hochwirksamen Eigenschaften zur Verfügung.

Anstelle einheitlicher Verbindungen können

auch Gemische, gegebenenfalls auch Abmischungen mit anderen Mikrobioziden, eingesetzt werden.

Die erfindungsgemäßen Verbindungen der Formel I können auch als mikrobizide Zusätze bei der Formulierung von Reinigungsmitteln in Kombination mit üblichen anionischen, nichtioniscüen, kationischen und amphoteren Tensiden dienen.

Dafür geeignete anionische Tenside sind beispielsweise Seife, Fettalkoholsulfate, Alkylethersulfate, Fettsäurekondensationsprodukte wie Tauride, Methyltauride, Sarkoside, ferner-α-Olefinsulfonate, Hydroxyalkansulfonate, sekundäre Alkansulfonate, Amidethersulfate oder Alkylbenzolsulfonate. Als nicht-ionische Tenside können beispielsweise Polyglykolmonoalkylether und -monoester, Aminoxide und Ethylenoxid-Propylenoxid-Kondensationsprodukte verwendet werden. Daneben ist auch die Kombination mit anderen amphoteren Tensiden wie Alkyl-Betainen, Alkylamido-Betainen, Imidäzolinderiväten oder Sulfobetainen möglich. Scbließlich können die erfindungsgemäßen Verbindungen der Formel I auch in Abmischung mit kationischen Tensiden wie Cetyltrimethylammoiiumchlorid, Cetyltrimethylammoniumbromid, Cetyldimethylbenzylammoniumchlorid, Didecyldimethylammoniumchlorid, Pentaoxyethylstearylammoniumchlorid, quaternierten Etheraminen oder polymeren quartären Ammoniumverbindungen eingesetzt werden. Bevorzugt sind nicht-ionische Tenside. Weitere Zusätze, die auf sonst übliche Weise in Reinigungsmitteln verwendet werden, können mit den erfindungsgemäßen Verbindungen der Formel I gegebenenfalls kombiniert werden. Dies sind beispielsweise viskositätserhöhende oder -erniedrigende Verbindungen wie Celluloseether, Elektrolyte wie beispielsweise Natriumchlorid oder Ammoniumchlorid, Fettsäurepolyglykolester, Alkanolamide, Magnesium-Aluminium-Silicate, Polyglykole, Glycerin und Ethanol.

Bei der Verarbeitung zu pulverförmigen Präparaten können weiterhin üblicherweise benutzte Füll- und Trägersubstanzen wie hochdisperse, amorphe Kieselsäure, Natriumsulfat, Magnesium-Aluminium-Silicat, Stärkederivate und dergleichen verwendet werden.

Ferner sind übliche Zusätze Bleichmittel,Chlorabspalter, Chelatbildner und gegebenenfalls auch Kunststoffdispersionen.

In solchen Reinigungsmitteln sind die erfindungsgemäßen Verbindungen der Formel I oder deren Gemische üblicherweise in einem Anteil von 1 bis 40, vorzugsweise von 10 bis 25 Gew.-%, wobei diese Mengen aber bei speziellen Anwendungszwecken auch über- oder unterschritten werden können.

Die folgenden Beispiele sollen die Erfindung näher erläutern:

**Beispiel 1**

In einem 2-1-Vierhalskolben mit Rückfluß, Thermometer, Rührer und Dosiergefäß werden67O g Cocosfettamin (Zusammensetzung in Mol-% bezüglich der Alkylreste R: $C_8$ 6 %, $C_{10}$ 6 %, $C_{12}$ 54 %, $C_{14}$ 18 %, $C_{16}$ 8 %, $C_{18}$ 8 %), 68 g Wasser, 34 g Methanol und 14 g konzentrierte Essigsäure auf 60 °C erhitzt. Innerhalb einer Stunde tropft man 373 g Acrylnitril zu und rührt weitere 24 h bei 75 °C unter Rückfluß. Anschließend wird mit 13 g NaOH und 120 g Wasser neutralisicrt, das Waschwasser separiert und das Produkt im Vakuum vom Restwasser und Lösungsmittel befreit. Man erhält 1000 g Cocosfettamino-di-propionitril (Ausbeute 95,9 %).

Ein 5-1-Autoklav wird mit 2020 g Cocosfettamino-dipropionitril, 3 g Kobalt-Trägerkontakt (Träger: Kieselgur) und 300 ml flüssigen Ammoniakj beschickt. Die Hydrierung erfolgt bei 150 bis 180 bar Wasserstoff-Druck und 110 bis 140 °C innerhalb von 3 Stunden. Nach Abfiltrieren des Katalysators erhält man 2010 g Produkt, das 85 bis 95 % Bis(3-aminopropyl)-cocosfettamin enthält. In einem 1-1-Vierhalskolben mit Thermometer, Rührer, Rückfluß und Dosiergefäß werden bei Raumtemperatur 108,7 g (0,33 mol) Bis-(3-aminopropyl)-cocosfettamin und 25,5 g Isopropanol vorgelegt und unter Rühren auf 70 °C erwärmt. Bei dieser Temperatur werden 40,1 g (0,66 mol) Eisessig zugetropft. Nach einer 3O minütigen Nachreaktion werden 58,8 g einer 50 gew.-%igen wäßrigen Cyanamid-Lösung (entsprechendO,69 mol Cyanamid) zugetropft, anschließend wird 3 h bei 80 °C nachgerührt, bis mit $AgNO_3$ keine freien Cyanid-Ionen mehr nachweisbar sind. Nach Abzug des Lösungsmittels und Trocknen bis zur Gewichtskonstanz beträgt die Ausbeute 99,5 % der Theorie. Charakteristische IR-Bande: ν(C=N) 1654 $cm^{-1}$.

**Beispiel 2**

131,2 g N,N-Bis-(3-aminopropyl)-stearylamin (0,33 mol) werden in 50,2 g Isobutanol auf 70 °C erwärmt, mit 40,1 g (0,66 mol) Eisessig versetzt, 58,8 g einer 50 gew.-%igen wäßrigen Lösung von Cyanamid (entsprechend 0,69 mol Cyanamid) zugetropft und 3 h nachgerührt. Ausbeute an kristallisierter und getrockneter Substanz 99,2 %. Charakteristische IR-Bande: ν(C=N) 1649 $cm^{-1}$.

**Beispiel 3**

95,5 g N,N-Bis-(3-aminopropyl)-decylamin werden in 21,6 g Isopropanol auf 70 °C erwärmt, mit 32,8 g Phosphorsäure (50 gew.-%ig in Wasser) versetzt und 58,8 g einer 50 gew.-%igen wäßrigen Lösung von Cyanamid (entsprechend

0,69 mol Cyanamid) zugetropft und 3 h nachgerührt. Ausbeute an kristallisierter und getrockneter Substanz 97,3 %. Charakteristische IR-Bande ν(C=N) 1652 $cm^{-1}$.

**Beispiel 4**

112,7 g N,N-Bis-(3-aminopropyl)-tridecylamin (0,33 mol) werden in 23,5 g Isobutanol auf 70 °C erwärmt, mit 40,1 g (0,66 mol) Eisessig versetzt und 58,8 g einer 50 gew.-%igen wäßrigen Lösung von Cyanamid (entsprechend 0,69 mol Cyanamid) zugetropft und 3 h nachgerührt. Ausbeute an kristallisierter und getrockneter Substanz 99,7 %. Charakteristische IR-Bande ν(C=N) 1658 $cm^{-1}$.

Die IR-Spektren werden gemessen an KBr-Preßlingen mit einem IR-Spektrometer Perkin Elmer 297.

Die mikrobiozide Wirkung der erfindungsgemäßen Verbindungen zeigt Tabelle I (µg Wirkstoff/ml Wasser; Kontaktzeit 24 und 48 h; Raumtemperatur). Die angegebenen Zahlen bedeuten die minimalen Hemmkonzentrationen, um die vorgelegte Keimzahl von $10^6$ Keimen pro ml abzutöten.

**TABELLE**

| | Verbindung aus Beispiel 1 | | Verbindung aus Beispiel 3 | | Verbindung aus Beispiel 4 | |
|---|---|---|---|---|---|---|
| Mikroorganismen | 24 h | 48 h | 24 h | 48 h | 24 h | 48 h |
| Staphylococcus aureus | 31,2 | 31,2 | 250 | 62,5 | 62,5 | 15,6 |
| Escherichia coli | 125 | 15,6 | 250 | 62,5 | 125 | 7,8 |
| Pseudomonas aeruginosa | 125 | 15,6 | 500 | 62,5 | 125 | 15,6 |
| Proteus mirabilis | 125 | 31,2 | 250 | 62,5 | 250 | 62,5 |
| Klebsiella pneumoniae | 125 | 31,2 | 500 | 62,5 | 125 | 15,6 |
| Aspergillus niger | >1000 | >1000 | >1000 | >1000 | >1000 | >1000 |
| Candida albicans | 500 | 250 | 250 | 62,5 | 250 | 62,5 |
| Desulfovibrio desulfuricans 85 | <3,9 | <3,9 | 7,8 | 7,8 | <3,9 | <3,9 |
| Desulfovibrio desulfuricans 39 | 7,8 | <3,9 | 15,6 | 7,8 | <3,9 | <3,9 |
| Algizide Wirkung | 7,8 | | 31,2 | | 31,2 | |

**Patentansprüche** für die Vertragsraaten BE, CH, DE, FR, GB, IT, LI, NL, SE

**Patentanspüche**

1. Salze von Bis-Guanidin-Drivaten der der allgemeinen Formel

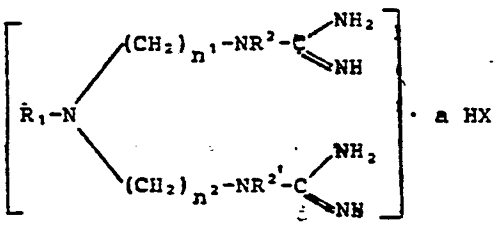

(I),

worin $R^1$ einen Alkylrest von 8 bis 18 C-Atomen, einen olefinisch ungesattigten Kohlenwasserstoff-Rest der gleichen Kettenlänge und mit 1 bis 3 Doppel1bindungen, einen oxalkylierten Alkylrest der Formel $R^{11}(OCHR^{1'}CH_2)_m$ worin $R^{11}$ die gleiche Bedeutung wie $R^1$ hat und in der m (als Mittelwert)eine Zahlvon 1 bis 10 bedeutet,$R^1$ H oder $CH_3$ ist und bei Werten von m 1 auch beide Bedeutungen haben kann, worin ferner $R^2$ und $R^{2'}$, gleich oder verschieden, Wasserstoff, einen Alkylrest mit 1 bis 3 C-Atomen oder einen Hydroxyalkylrest mit 1 bis 3 C-Atomen bedeuten, $n^1$ und $n^2$, gleich oder verschieden, die Zahlen 2 oder 3 sind und HX eine zur Salzbildung mit dem Bis-Guanidin befähigte Säure sowie a eine Zahl von 1 bis 3 ist.

2. Verfahren zur Herstellung von Verbindungen der Formel I gemäß Anspruch 1, dadurch gekennzeichnet, daß ein Amin der Formel

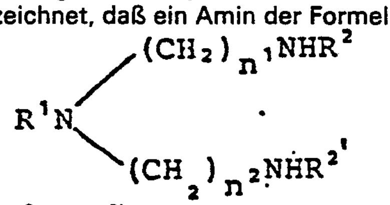

in der $R^2$ und $R^{2'}$, gleich oder verschieden, Wasserstoff, einen Alkyl- oder einen Hydroxyalkylrest mit je 1 bis 3 C-Atomen bedeuten und $R^1$ die in Anspruch 1 definierte Bedeutung hat, mit 2 mol Cyanamid oder mit 1 mol Dicyanamid sowie mit einer zur Salzbildung mit dem sich bildenden Bis-Guanidin befähigten Säure HX, deren eingesetzte Molmenge sich ergibt aus dem angestrebten Protonierungsgrad a des Bis-Guanidins, geteilt durch die Basizität der Säure HX, umgesetzt wird.

3. Mikrobizide Zubereitung, enthaltendbis zu 90 Gew.-% mindestens einer Verbindung der Formel I gemäß Anspruch 1 und Wasser oder Alkanole mit 1 bis 6 C-Atomen oder Mischungen dieser beiden Lösungsmittel.

4. Mikrobizides Reinigungsmittel, enthaltend Wasser, Alkohol oder Gemische dieser beiden Lösungsmittel sowie mindestens ein nichtionisches, anionisches, kationisches oder amphoteres Tensid sowie gegebenenfalls sonstige in Reinigungsmitteln übliche Zusätze, gekennzeichnet durch einen Gehalt an einer wirksamen Menge mindestens einer Verbindung der Formel I gemäß Anspruch 1.

5. Verwendung der Verbindungen der Formel I gemäß Anspruch 1, gegebenenfalls im Gemisch mit anderen Mikrobiziden zur Bekämpfung von sulfatreduzierenden Bakterien bei der Sekundärförderung aus Erdöl-Lagerstätten.

**Patentansprüche** für den Vertragsstaat AT

1. Verfahren zur Herstellung von Salzen von Bis-GuanidinDerivaten der allgemeinen Formel

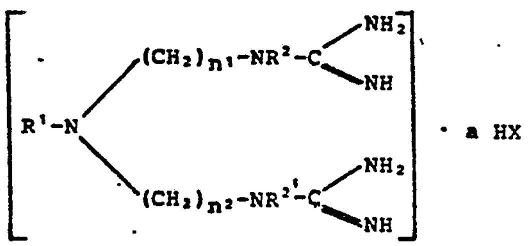

(I),

worin $R^1$ einen Alkylrest von 8 bis 18 C-Atomen, einen olefinisch ungesättigten Kohlenwasserstoff-Rest der gleichen Kettenlänge und mit 1 bis 3 Doppelbindungen, einen oxalkylierten Alkylrest der Formel $R^{11}(OCHR^{1'}CH_2)_m$ worin $R^{11}$die gleiche Bedeutung wie $R^1$ hat und in der m (als Mittelwert) eine Zahl von 1 bis 10 bedeutet, $R^1$ H oder $CH_3$ ist und bei Werten von m Z1 auch beide Bedeutungen haben kann, worin ferner $R^2$ und $R^{2'}$, gleich oder verschieden, Wasserstoff, einen Alkylrest mit 1 bis 3 C-Atomen oder einen Hydroxyalkylrest mit 1 bis 3 C-Atomen bedeuten, $n^1$ und $n^2$, gleich oder verschieden, die Zahlen 2 oder 3 sind und HX eine zur Salzbildung mit dem Bis-Guanidin befähigte Säure sowie a eine Zahl von 1 bis 3 ist, dadurch gekennzeichnet, daß ein Amin der Formel

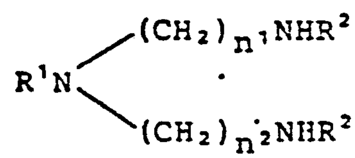

In der $R^2$ und $R^2$ , gleich oder verschieden, Wasserstoff, einen Alkyl- oder einen Hydroxyalkylrest mit je 1 bis 3 C-Atomen bedeuten und $R^1$ die oben definierte Bedeutung hat, mit 2 mol Cyanamid oder mit 1 mol Dicyanamid sowie mit einer zur Salzbildung mit dem sich bildenden Bis-Guanidin befähigten Säure HX deren eingesetzte Molmenge sich ergibt aus dem angestrebten Protonierungsgrad a des Bis-Guanidins, geteilt durch die Basizität der Säure HX, umgesetzt wird.

2. Mikrobizide Zubereitung, enthaltend bis zu 90 Gew.-% mindestens einer Verbindung der Formel I gemäß Anspruch 1 und Wasser oder Alkanole mit 1 bis 6 C-Atomen oder Mischungen dieser

beiden Lösungsmittel.

3. Mikrobizides Reinigungsmittel, enthaltend Wasser, Alkohol oder Gemische dieser beiden Lösungsmittel sowie mindestens ein nichtionisches, anionisches, kationisches oder amphoteres Tensid sowie gegebenenfalls sonstige in Reinigungsmitteln übliche Zusätze, gekennzeichnet durch einen Gehalt an einer wirksamen Menge mindestens einer Verbindung der Formel I gemäß Anspruch 1.

4. Verwendung der Verbindungen der Formel I gemäß Anspruch 1, gegebenenfalls im Gemisch mit anderen Mikrobiziden, zur Bekämpfung von sulfatreduzierenden Bakterien bei der Sekundärförderung aus Erdöl-LagerStätten.

**Claims** for the Contraicting states : BE, CH, DE, FR, GB, IT, LI,NL, SE

1. A salt of a bisguanidine derivative of the formula

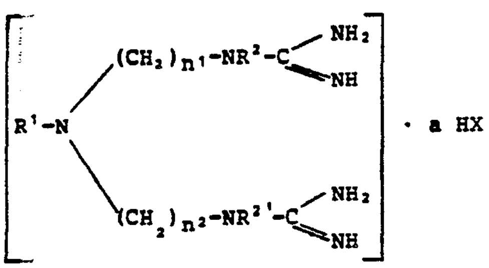

(I),

in which $R^1$ denotes an alkyl radical having 8 to 18 carbon atoms, an olefinic unsaturated hydrocarbon radical of the same chain length and having 1 to 3 double bonds, or an oxalkylated alkyl radical of the formula $R^{11}$ $(OCHR^1\ CH_2)m$, in which $R^{11}$ has the same meaning as $R^1$ and in which m (as the mean value) denotes a number from 1 to 10, and $R^1$ is H or $CH_3$ and, for values of m greater than 1, can also have both meanings, and in which $R^2$ and $R^2$ , being identical or different, denote hydrogen, an alkyl radical having 1 to 3 carbon atoms or a hydroxyalkyl radical having 1 to 3 carbon atoms, $n^1$ and $n^2$, being identical or different, are the numbers 2 or 3, and HX is an acid able to form a salt with the bisguanidine,and a is a number from 1 to 3.

2. A process for the preparation of a compound of the formula I as claimed in claim 1, which comprises reacting an amine of the formula

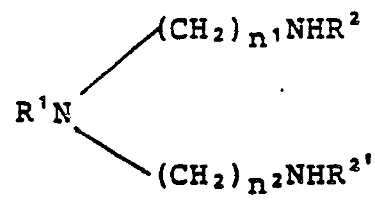

in which $R^2$and $R^2$ , being identical or different, denote hydrogen, an alkyl or a hydroxyalkyl radical each having 1 to 3 carbon atoms, and $R^1$ has the meaning defined in claim 1, with 2 mol of cyanamide or with 1 mol of dicyanamide and with an acid HX which is able to form a salt with the bisguanidine which is formed, the molar amount of which acid employed resulting from the intended degree of protonation a of the bisguanidine divided by the basicity of the acid HX.

3. A microbicidal formulation containing up to 90 % by weight of at least one compound of the formula I as claimed in claim 1, and water or alkanols having 1 to 6 carbon atoms, or mixtures of these two solvents.

4. A microbicidal cleaning agent, containing water, alcohol or mixtures of these two solvents, and at least one non-ionic, anionic, cationic or amphoteric surfactant and, where appropriäte, other additives customarily used in cleaning agents, which agent contains an effective amount of at least one compound of the formula I as claimed in claim 1.

5. The use of the compounds of the formula I as claimed in claim 1, if desired in admixture with other microbicides, for controlling sulfate-reducing bacteria in the secondary recovery from petroleum reservoirs.

**Claims** for the contracting state: AT

1. A process for the preparation of salts of bisguanidine derivatives of the formula

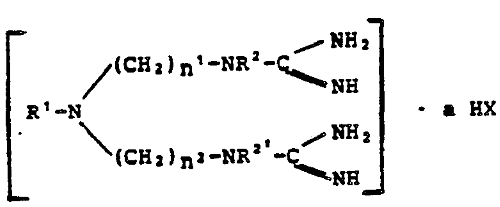

(I),

in which $R^1$ denotes an alkyl radical having 8 to 18 carbon atoms, an olefinic unsaturated hydrocarbon radical of the same chain length and having 1 to 3 double bonds, or an oxalkylated alkyl radical of the formula $R^{11}(OCHR^{1'}\ CH_2)m$, in which $R^{11}$ has the same meaning as $R^1$ and in which m (as the meanvalue) denotes a number from 1 to 10, and $R^{1'}$is H or $CH_3$ and, for values of m greater than 1, can also have both meanings,

and in which $R^2$ and $R^{2'}$, being identical or different, denote hydrogen, an alkyl radical having 1 to 3 carbon atoms or a hydröxyalkyl radical having 1 to 3 carbon atoms, $n^1$ and $n^2$, being identical or different, are the numbers 2 or 3, and HX is an acid able to form a salt with the bisguanidine, and a is a number from 1 to 3 which comprises reacting an amine of the formula

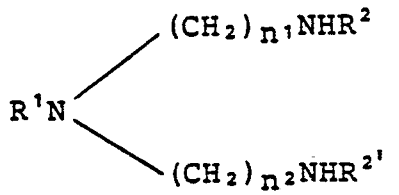

in which $R^2$ and $R^{2'}$, being identical or different, denote hydrogen, an alkyl or a hydroxyalkyl radical each having 1 to 3 carbon atoms, and $R^1$ has the meaning defined above with 2 mol of cyanamide or with 1 mol of dicyanamide and with an acid HX which is able to form a salt with the bisguanidine which is formed, the molar amount of which acid employed resulting from the intended degree of protonation a of the bisguanidine divided by the basicity of the acid HX.

2. A microbicidal formulation containing up to 90 % by weight of at least one compound of the formula I as claimed in claim 1, and water or alkanols having 1 to 6 carbon atoms, or mixtures of these two solvents.

3. A microbicidal cleaning agent, containing water, alcohol or mixtures of these two solvents, and at least one non-ionic, anionic, cationic or amphoteric surfactant and, where appropriate, other additives customarily used in cleaning agents, which agent contains an effective amount of at least one compound of the formula I as claimed in claim 1.

4. The use of the compounds of the formula I as claimed in claim 1, if desired in admixture with other microbicides, for controlling sulfate-reducing bacteria in the secondary recovery from petroleum reservoirs.

**Revendications** pour les Etats contractans: BE, CH, DE, FR, GB, IT, LI, NL, SE

1. Sels de bis-guanidines répondant à la formule générale I :

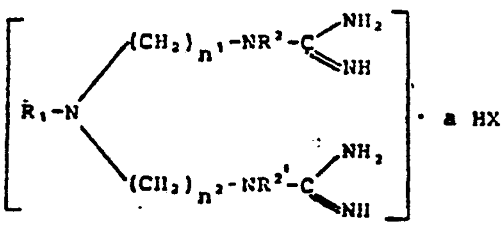

(I)

dans laquelle

$R^1$ représente un radical alkyle contenant de 8 à 18 atomes de carbone, un radical hydrocarboné éthylénique ayant la même longueur de chaine et contenant de 1 à 3 doubles liaisons, un radical alkyle alcoxylé de formule $R^{11}$ $(OCHR^1 CH_2)_m$ [dans ce radical $R_{11}$ a la même signification que $R^{1'}$ m (en tant que valeur moyenne) représente un nombre de 1 à 10, et $R^1$ représente H ou $CH_3$ et, pour des valeurs de m supérieures à2 1, peut prendre les deux, significations-].

$R^2$ et $R^2$ représentent chacun, indépendamment l'un de l'autre, l'hydrogène, un alkyle contenant de 1 à 3 atomes de carbone ou un hydroxyalkyle contenant de 1 à 3 atomes de carbone,

$n^1$ et $n^2$ sont égaux chacun, indépendamment l'un de l'autre, à 2 ou à 3,

HX représente un acide apte à former un sel avec la bis-guanidine et

a représente un nombre de 1 à 3.

2. Procédé de préparation de composés de formule I selon la revendication 1, procédé caractérisé en ce qu'on fait réagir une amine répondant à la formule suivante:

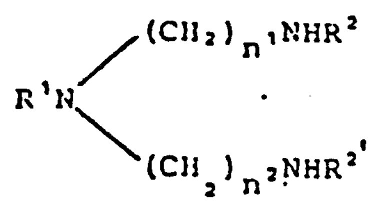

dans laquelle $R^2$ et $R^{2'}$ représentent chacun, indépendamment l'un de l'autre, l'hydrogène, un radical alkyle ou un radical hydroxyalkyle contenant chacun de 1 à 3 atomes de carbone, et $R^1$ a la signification donnée à la revendication 1, avec 2 mol de cyanamide ou avec 1 mol de cyanoguanidine ainsi qu'avec un acide HX apte à former un sel avec la bisguanidine qui se forme, acide qui est mis en jeu en une quantité, en moles, égale au quotient du degré de protonation a voulu pour la bis-guanidine par la fonctionnalité acide de l'acide HX.

3. Composition microbicide contenant jusqu'à 90 % en poids d'au moins un composé de formule I selon la revendication 1 ainsi que de l'eau ou des alcanols en $C_1$ à $C_6$ ou des mélanges de ces deux solvants.

4. Produit de nettoyage microbicide contenant de l'eau, un alcool ou un mélange de ces deux

solvants, ainsi qu'au moins un surfactif non ionique, anionique, cationique ou amphotère et éventuellement d'autres additifs usuels dans des produits de ce genre, ledit produit de nettoyage étant caractérisé en ce qu'il contient une quantité efficace d'au moins un composé de formule I selon la revendication 1.

5. Application des composés de formule selon la revendication 1, éventuellement en mélange avec d'autres microbicides, pour la lutte contre des bactéries réductrices de sulfates dans l'exploitation secondaire de gisements de pétrole.

**Revendications** pour l'Etat contractant: AT

1. Procédé de préparation de sels de bisguanidines répondant à la formule générale I:

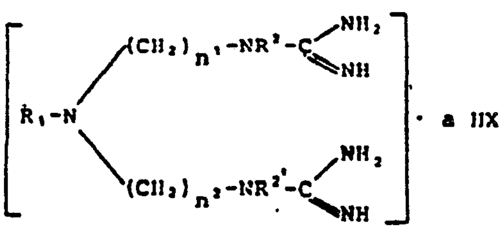

(I)

dans laquelle

$R^1$ représente un radical alkyle contenant de 8 à 18 atomes de carbone, un radical hydrocarboné éthylénique ayant la meme longueur de chaine et contenant de 1 à 3 doubles liaisons, un radical alkyle alcoxylé de formule $R^{11}$ ($OCHR^1$ $CH_2)_m$ [dans ce radical R a la même signification que $R^1$, m (en tant que valeur moyenne) représente un nombre de 1 à 10, et $R^{1'}$ représente H ou $CH_3$ et, pour des valeurs de m supérieures à 1, peut prendre les deux significations],

$R^2$ et $R^2$ représentent chacun, indépendamment l'un de l'autre, l'hydrogène, un alkyle contenant de 1 à3 atomes de carbone ou un hydroxyalkyle contenant de 1 à 3 atomes de carbone,

$n^1$ et $n^2$ sont égaux-chacun, indépendamment l'un de l'autre, à 2 ou à 3,

HX représente un acide apte à former un sel avec la bis-guanidine et

a représente un nombre de 1 à 3, procédé caractérisé en ce qu'on fait réagir une amine répondant à la formule:

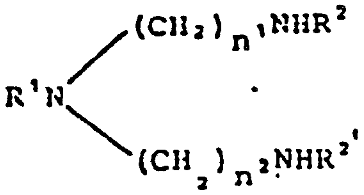

dans laquelle $R^2$ et $R^{2'}$ représentent chacun, l'un de l'autre, l'hydrogène, un radical alkyle ou un radical hydroxyalkyle contenant chacun de 1 à 3 atomes de carbone, et $R^1$ a la signification indiquée ci-dessus, avec 2 mol de cyanamide ou avec 1 mol de cyanoguanidine ainsi qu'avec un acide HX apte à former un sel avec la bis-guanidine qui se forme, acide qui est mis en jeu en une quantité, en moles, égale au quotient du degré de protonation a voulu pour la bis-guanidine par la fonctionnalité acide de l'acide HX.

2. Composition microbicide contenant jusqu'à 90 % en poids d'au moins un composé de formule I selon la revendication 1 ainsi que de l'eau ou des alcanols en $C_1$ à $C_6$ ou des mélanges de ces deux solvants.

3. Produit de nettoyage microbicide contenant de l'eau, un alcool ou un mélange de ces deux solvants, ainsi qu'au moins un surfactif non ionique, anionique, cationique ou amphotère et éventuellement d'autres additifs usuels dans des produits de ce genre, ledit produit de nettoyage étant caractérisé en ce qu'il contient une quantité efficace d'au moins un composé de formule I selon la revendication 1.

4. Application des composés de formule I selon la revendication 1, éventuellement en mélange avec d'autres microbicides, pour la lutte contre des bactéries réductrices de sulfates dans l'exploitation secondaire de gisements de pétrole.